# EUROPEAN PATENT APPLICATION

(11) **EP 2 439 320 A2**
(43) Date of publication of application: **11.04.2012**
(21) Application number: 10783592.8
(22) Date of filing: 03.06.2010
(51) Int. Cl.: D03D 25/00, D06M 10/06, B32B 27/14, D06M 17/04

(54) **FABRIC AND FABRIC PRODUCT PROVIDED WITH SCORIA MOLDED BODY, AND METHOD FOR ADHERING SCORIA**

(30) Priority: 03.06.2009 KR 20090049255
(71) Applicant: Baek, Woo Hyun, Gyeonggi-do 442-831 (KR); Baek, Sang Yeop, Gyeonggi-do 442-831 (KR)
(72) Inventor: Baek, Woo Hyun, Gyeonggi-do 442-831 (KR); Baek, Sang Yeop, Gyeonggi-do 442-831 (KR)
(74) Representative: Ricker, Mathias
(86) International application number: PCT/KR2010/003560
(87) International publication number: WO 2010/140843

(57) **Abstract**

The present invention relates to fabric and a fabric product provided with a scoria molded body which brings a scoria molded body containing scoria powder into contact with the human body to activate the function of the human body, thereby promoting public health, and a method for adhering scoria. The fabric provided with the scoria molded body according to the present invention is **characterized in that** the fabric has a scoria molded body placed between a first fabric and a second fabric, and the scoria molded body is formed by mixing scoria powder containing silicon dioxide and titanium dioxide with water, molding and solidifying the mixture, and has 90% or more of far infrared ray radiation characteristics and rotating electromagnetic wave characteristics. In addition, the fabric of the present invention can be applied to fabric products that could come in contact with the human body, for example, a brassiere or briefs. Therefore, a user can be easily provided with far infrared ray radiation characteristics and rotating electromagnetic wave characteristics through a scoria component by bringing the scoria molded body of the present invention in contact with the human body.

## Description

### [Technical Field]

The present invention relates to a fabric and a fabric product provided with a scoria molded body, which is brought into contact with a human body to activate the function of the human body, thereby promoting public health, and a method for adhering scoria.

### [Background Art]

Recently, environmental pollution has become serious, people's dietary habits have changed, and many people have been exposed to various electromagnetic environments. Thus, the risk of various diseases such as adult diseases has increased.

Certain diseases whose incidence increases in the aged over forties are collectively referred to as adult diseases, and basically, certain diseases occurring in those who get old are referred to as adult diseases.

However, the age of the onset of adult diseases has decreased due to various problems such as changes in modern society such as westernization of dietary habits, popularity of instant foods, excessive stress, lack of exercise, etc. and environmental pollution such as air pollution, automobile exhaust gas, water pollution, etc.

The adult diseases occur without any particular symptoms in the earliest stage and, as the diseases get worse, they have various symptoms and cause various complications in serious cases. General symptoms of adult diseases include tiredness, dizziness, temporary blindness, neck stiffness, numbness or tingling, feeling of heaviness, shortness of breath, rash, fever, etc., and in serious cases, stroke, heart disease, liver disease, etc. That is, adult diseases such as hypertension, diabetes, arteriosclerosis, obesity, hyperlipidemia, etc. do not directly affect the life. However, when these diseases cause complications, they lead to stroke, cancers, malignant tumors, heart disease, liver diseases, thereby significantly affecting the life.

Therefore, people should continue their efforts to protect themselves from adult diseases in order to maintain healthy lives. That is, people should continue their efforts to improve dietary habits, to perform exercises, and to escape from factors such as stress, environmental pollution, etc., which accelerate the aging of body cells.

However, it is very difficult to continue their efforts to maintain healthy lives without their strong convictions.

### [Disclosure]

### [Technical Problem]

Therefore, the present invention has been made in consideration of the above-described circumstances, and a technical object of the present invention is to provide a fabric and a fabric product provided with a scoria molded body and a method for adhering scoria, which can provide various functions of scoria to a user by bringing a scoria molded body containing scoria powder having excellent far-infrared radiation characteristics and rotating electromagnetic wave characteristics into contact with the user' body.

### [Technical Solution]

In a first aspect, the present invention provides a fabric comprising a scoria molded body disposed between a first fabric sheet and a second fabric sheet, wherein the scoria molded body is formed by mixing scoria powder containing silicon oxide and titanium oxide with water and molding and solidifying the mixture and has a far-infrared radiation rate of 90% or higher and rotating electromagnetic wave characteristics.

In the present invention, the fabric is attached to a wear product worn on a human body.

In the present invention, the fabric is attached to and detached from the wear product.

In a second aspect, the present invention provides a product comprising a scoria molded body disposed between a lining and an outer fabric, wherein the scoria molded body is formed by mixing scoria powder containing silicon oxide and titanium oxide with water and molding and solidifying the mixture, has a far-infrared radiation rate of 90% or higher and rotating electromagnetic wave characteristics, and is disposed on portions corresponding to the front and rear of a human body.

In a third aspect, the present invention provides a fabric product comprising a scoria molded body disposed between a lining and an outer fabric of a brassiere, wherein the scoria molded body is formed by mixing scoria powder containing silicon oxide and titanium oxide with water and molding and solidifying the mixture and has a far-infrared radiation rate of 90% or higher and rotating electromagnetic wave characteristics.

In the present invention, the scoria molded body is disposed on a pair of brassiere cups and a brassiere strap.

In a fourth aspect, the present invention provides a fabric product comprising a scoria molded body disposed between a lining and an outer fabric of a pair of briefs, wherein the scoria molded body is formed by mixing scoria powder containing silicon oxide and titanium oxide with water and molding and solidifying the mixture and has a far-infrared radiation rate of 90% or higher and rotating electromagnetic wave characteristics.

In the present invention, the scoria molded body is disposed on a waist band and a perineal region.

In a fifth aspect, the present invention provides a method for adhering scoria, the method comprising: a first step of preparing a mixed solution by mixing scoria powder, which contains silicon oxide and titanium oxide with and has rotating electromagnetic wave characteristics, with an adhesive; and a second step of applying the mixed solution onto a fabric.

In the present invention, the mixed solution is applied to the fabric by screen printing.

### [Advantageous Effects]

According to the present invention, a user can easily experience far-infrared radiation effects and rotating electromagnetic wave effects of scoria in such a manner than a scoria molded body is brought into contact with the user's body, thereby promoting public health.

That is, the user can experience various effects due to activation of cell tissues and purification of the blood, such as prevention of adult diseases, discharge of bodily wastes, blood circulation, fatigue recovery, pain relief, sound sleep, prevention of cancer caused by electromagnetic waves, blocking of water veins, neutralization of metal toxicities and organic toxicities, treatment of atopic diseases, prevention of sick house syndrome, etc.

### [Description of Drawings]

FIG. 1 is a diagram illustrating the configuration of a fabric 100 provided with a scoria molded body according to the present invention.
FIGS. 2 to 12 are diagrams illustrating the test results of rotating electromagnetic wave characteristics of scoria that constitutes a scoria molded body 130 shown in FIG. 1.
FIGS. 13 and 14 are diagrams illustrating a fabric product provided with a scoria molded body according to the present invention.
FIG. 15 is a diagram illustrating the use of the fabric provided with a scoria molded body according to the present invention.

### Brief description of major components in the drawings:

100: fabric provided with scoria molded body
110: lining 120: outer fabric
130: scoria molded body 140: silicone adhesive

### [Mode for Invention]

Hereinafter, preferred embodiments in accordance with the present invention will be described with reference to the accompanying drawings. The preferred embodiments are provided so that those skilled in the art can sufficiently understand the present invention, but can be modified in various forms and the scope of the present invention is not limited to the preferred embodiments.

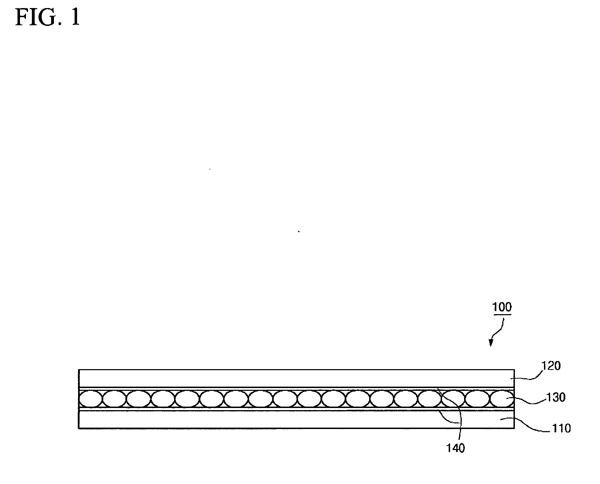

FIG. 1 is a diagram illustrating the configuration of a fabric 100 provided with a scoria molded body according to the present invention.

As shown in FIG. 1, a fabric 100 provided with a scoria molded body according to the present invention comprises a first fabric sheet 110, a second fabric sheet 120, and a plurality of scoria molded bodies 130 interposed between the first and second fabric sheets 110 and 120.

Moreover, the scoria molded bodies 130 are bonded between the first and second fabric sheets 110 and 120 using an adhesive material, more particularly, a silicone adhesive 140. The silicone adhesive 140 may be applied to either the first fabric sheet 110 or the second fabric sheet 120.

The scoria molded body 130 may have various shapes such as a circular shape, an oval shape, a quadrangular shape, etc. Preferably, the scoria molded body 130 may be formed with a plate shape to increase the contact area with the silicone adhesive 140 such that the scoria molded body 130 can be more stably bonded between the first and second fabric sheets 110 and 120.

That is, the fabric 100 provided with the scoria molded body 130 according to the present invention is formed as follows. The silicone adhesive 140 is applied to a predetermined upper portion of the first fabric sheet 110 and melted at a high temperature, and the scoria molded body 130 is adhered to the portion where the silicone adhesive is applied. Then, the silicone adhesive 140 is applied to the upper surface of the scoria molded body 130 and melted at a high temperature, and then the second fabric sheet 120 is adhered thereto. Here, the silicone adhesive 140 may preferably be made of a material harmless to the human body.

Next, a process of forming the scoria molded body 130 will be described.

First, a scoria rock is crushed into scoria powder, and the scoria powder is mixed with water in a predetermined ratio to make a scoria paste. The scoria paste is molded into a specific shape, for example, a plate shape using a molding machine (not shown). Here, the scoria molded body 130 may have various diameters such as 0.5, 1, 2, 3, 5, 10 mm, etc. Then, the molded scoria is placed in a dryer, for example, an electric heating device at about 700 to 1,200°C and flash-cooled to be solidified and to have porosity, thereby completing the scoria molded body 130.

Here, the scoria used to form the scoria molded body 130 means a rock that has lots of air holes, dark colors such as black, brown, red, etc., and a diameter of 4 mm or higher among volcanic ejecta, and is a weak alkaline resource having a pH of 7.2 to 7.8.

The scoria has the following performance and functions. First, as a result of the test performed by Korea Institute of Construction Material (KICM), it has been reported that the scoria has a high far-infrared radiation rate of 90% or higher, i.e., 93% and has antibacterial, purifying, and detoxification effects to prevent inhabitation and propagation of harmful bacteria or fungi. Far-infrared rays are radiated to molecules such as water, which constitute cells in the body, to activate cell tissues by vibration applied to subtly vibrate the cells about 2,000 times for 1 minute, thereby promoting vital activities. Moreover, the far-infrared rays function to generate heat energy in the body and to naturally discharge bodily wastes which are harmful substances that the cells in the body have. Furthermore, the far-infrared rays have an effect on the cells, the most basic components of the body, to stimulate blood circulation and activate self defense mechanisms of the body to recover one's health. In addition, the far-infrared rays provide functions such as promotion of perspiration, pain relief, removal of heavy metals, etc.

Moreover, according to the results of chemical analysis performed by Korea Institute of Construction Material, it has been reported that the scoria of the present invention has no harmful ingredients, is excellent at absorbing heavy metals such as mercury, lead, etc., and contains silicon dioxide (SiO₂), aluminum hydroxide (Al₂O₃), iron oxide (Fe₂O₃), calcium oxide (CaO), magnesium oxide (MgO), potassium oxide (K₂O), sodium oxide (Na₂O), and titanium oxide (TiO₂) as main ingredients. Here, the scoria contains 3.97% of titanium oxide (TiO₂) and has a sterilizing function that eliminates toxic substances. Moreover, the silicon dioxide (SiO₂) acts as an adsorbent, and the aluminum hydroxide (Al₂O₃), calcium oxide (CaO), and magnesium oxide (MgO) act as a coagulant and a neutralizing agent. The scoria also has a heavy metal adsorption rate 3 to 25 times higher depending on the type of heavy metal. The following table 1 shows the results of chemical analysis of scoria with respect to each color.

**[Table 1]**

| Color/ Ingredient | Silicon oxide | Aluminum oxide | Iron oxide | Titanium oxide | Calcium oxide | Magnesium oxide | Potassium oxide & Sodium oxide |
|---|---|---|---|---|---|---|---|
| Reddish brown | 45.40 | 16.87 | 14.92 | 3.97 | 6.95 | 6.13 | 4.06 |
| Yellowish brown | 45.70 | 19.87 | 15.55 | 2.92 | 4.41 | 6.64 | 1.77 |
| Gray | 46.22 | 16.26 | 11.66 | 2.35 | 5.09 | 7.91 | 4.28 |
| Black | 40.02 | 19.15 | 12.27 | 2.60 | 8.16 | 6.59 | 1.92 |

Furthermore, the scoria according to the present invention has the body's energy circulation i.e., rotating electromagnetic wave characteristics. Rotating electromagnetic waves, also called a torsion field (spin field), mean the rotation of the electron or nuclear with various spatial structures due to spiral rotation, not simple rotation. The rotating electromagnetic waves include right-hand circularly polarized positive electromagnetic waves, right-hand circularly polarized negative electromagnetic waves, left-hand circularly polarized positive electromagnetic waves, left-hand circularly polarized negative electromagnetic waves, left-hand circularly polarized positive/right-hand circularly polarized positive electromagnetic waves, left-hand circularly polarized positive/left-hand circularly polarized negative electromagnetic waves, right-hand circularly polarized positive/right-hand circularly polarized negative electromagnetic waves, and right-hand circularly polarized negative/left-hand circularly polarized negative electromagnetic waves. Among them, the scoria according to the present invention has the characteristics of the left-hand circularly polarized positive electromagnetic waves that neutralize metal toxicities and organic toxicities, the characteristics of the left-hand circularly polarized positive/right-hand circularly polarized positive electromagnetic waves and the left-hand circularly polarized positive/left-hand circularly polarized negative electromagnetic waves that are effective for the treatment of atopic diseases and the prevention of sick house syndrome. When these rotating electromagnetic waves penetrate the human body, the energy that interrupts the flow of meridian energy is expelled to the outside and the flow of meridian energy is normalized, thereby promoting immunization activities and biological activities of the body.

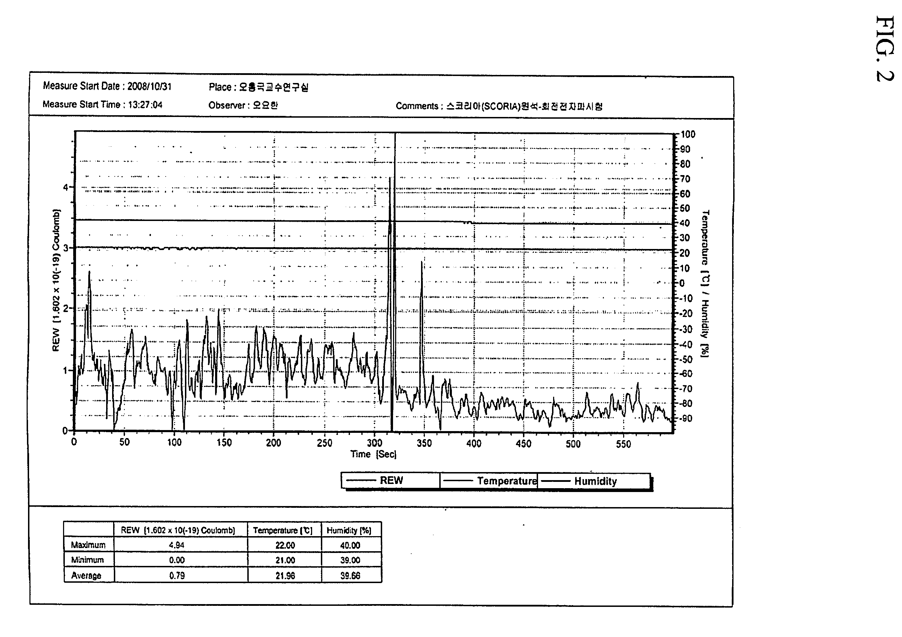

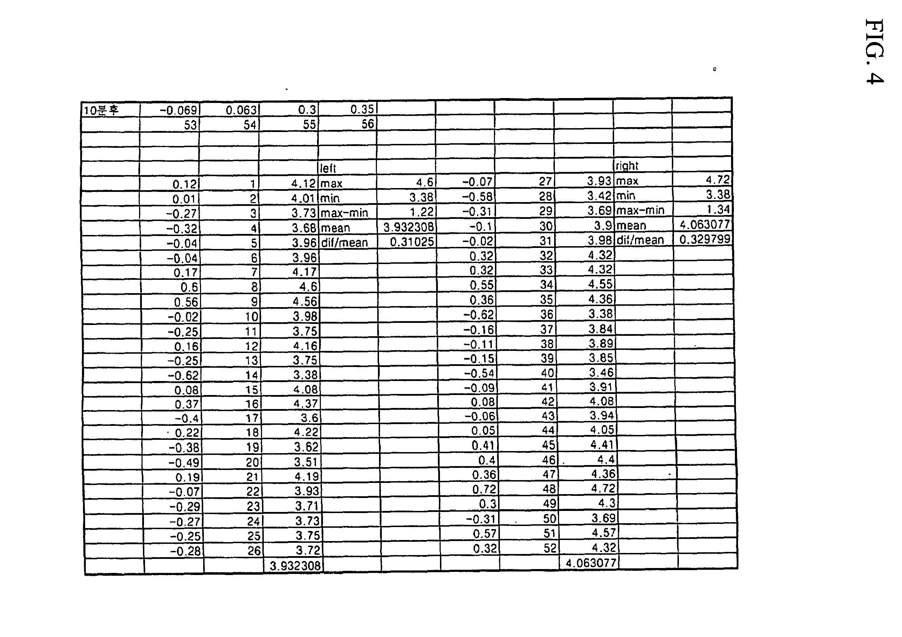

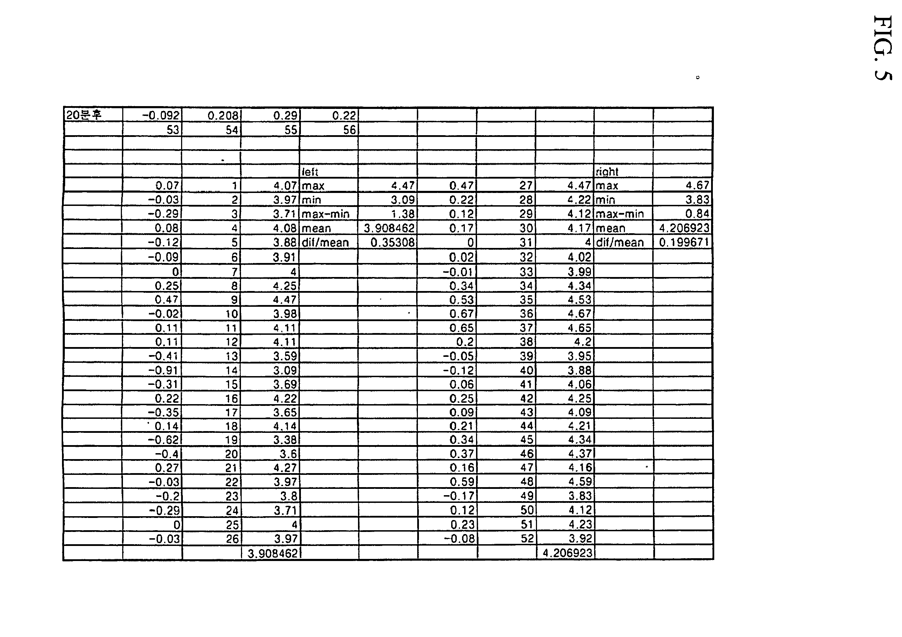

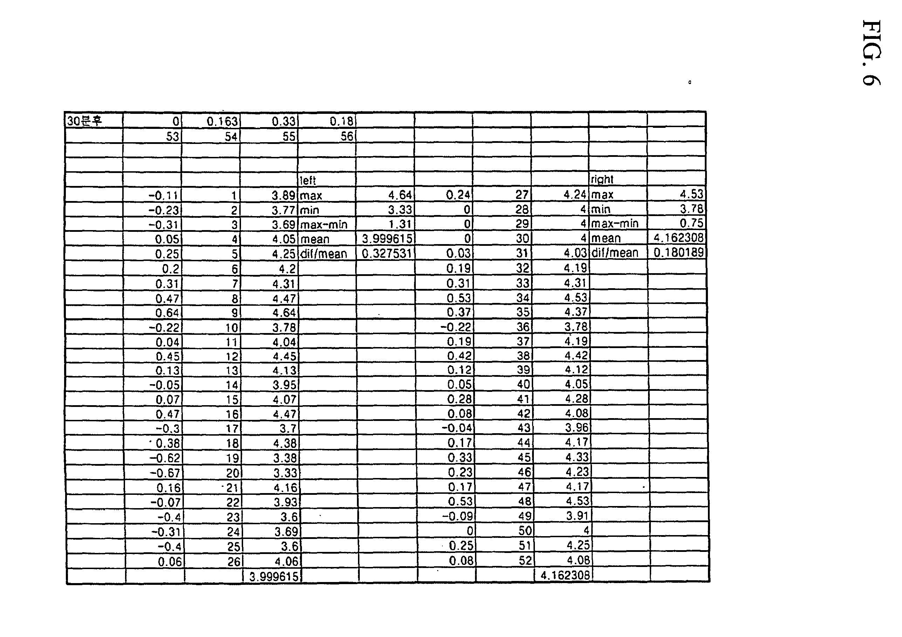

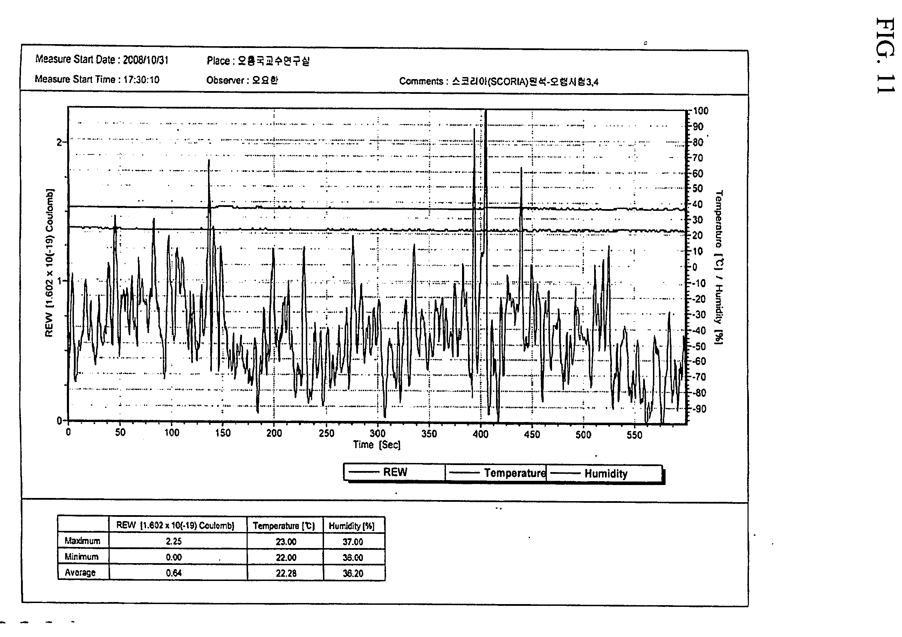

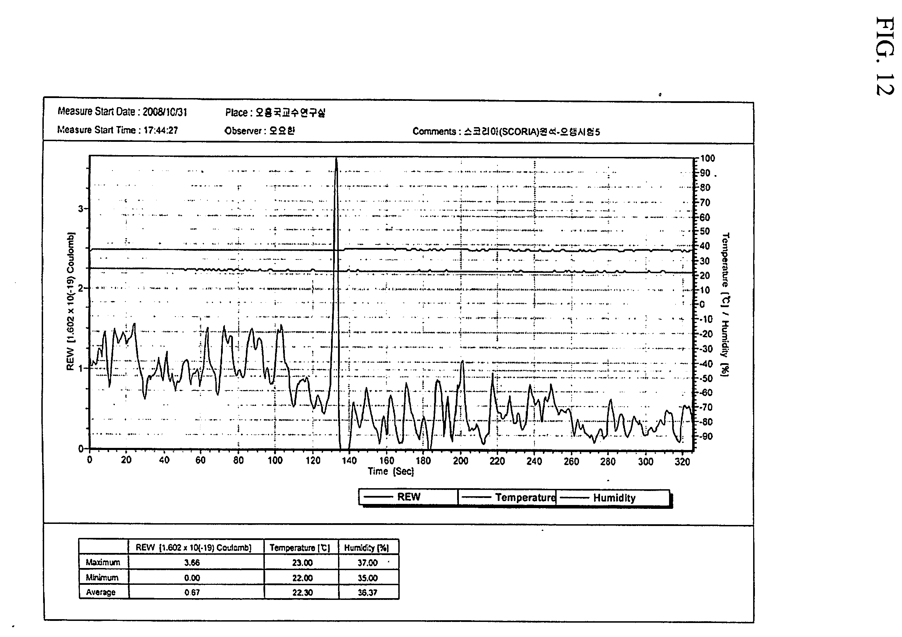

FIGS. 2 to 12 are diagrams illustrating the test results of rotating electromagnetic wave characteristics of scoria measured by Department of Mechanical Engineering at Ajou University and Electromagnetic Wave Measurement Team in Industrial-Academic Cooperation Association.

FIG. 2 shows the results of the measurement of rotating electromagnetic waves of scoria rock, in which the measurement results are calculated by subtracting the average value of the control group from the average value of the sample, from which it can be seen that the rotating electromagnetic waves are measured as the left-hand circularly polarized positive/left-hand circularly polarized negative electromagnetic waves.

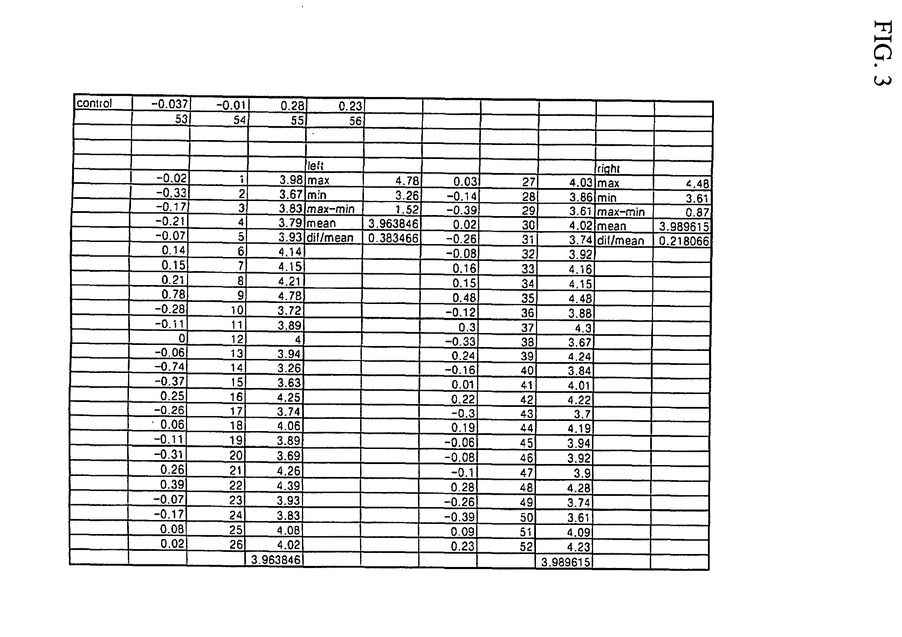

FIGS. 3 to 6 show the results of the measurement of the effect of scoria rock on meridian circulation. That is, FIGS. 3 to 6 show the amount of meridian energy flow measured every ten minutes before and after the use of scoria rock, from which it can be seen that the balanced flow of meridian energy is repeatedly destroyed and restored, which means that the rotating electromagnetic waves generated from the scoria rock penetrate the human body to continuously repeat the action of expelling the energy that interrupts the flow of meridian energy to the outside and normalizing the flow of meridian energy, thereby promoting immunization activities and biological activities of the body.

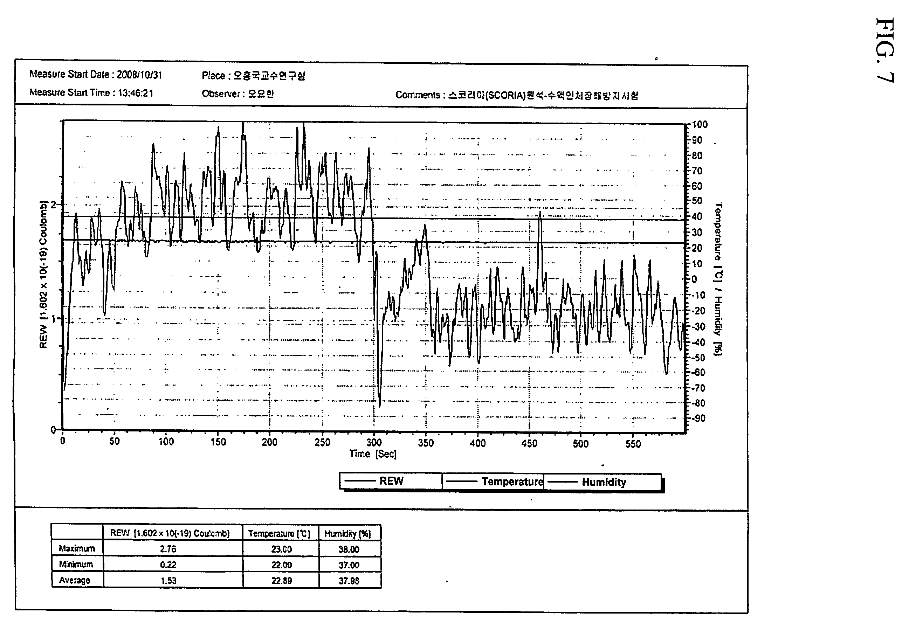

FIG. 7 shows the results of the test of the effect of scoria rock for preventing the influence of water vein to human body, in which harmful left-hand energy emitted from the water vein is removed by right-hand energy, from which it can be seen that the effect of scoria rock for preventing the influence of water vein to human body is excellent.

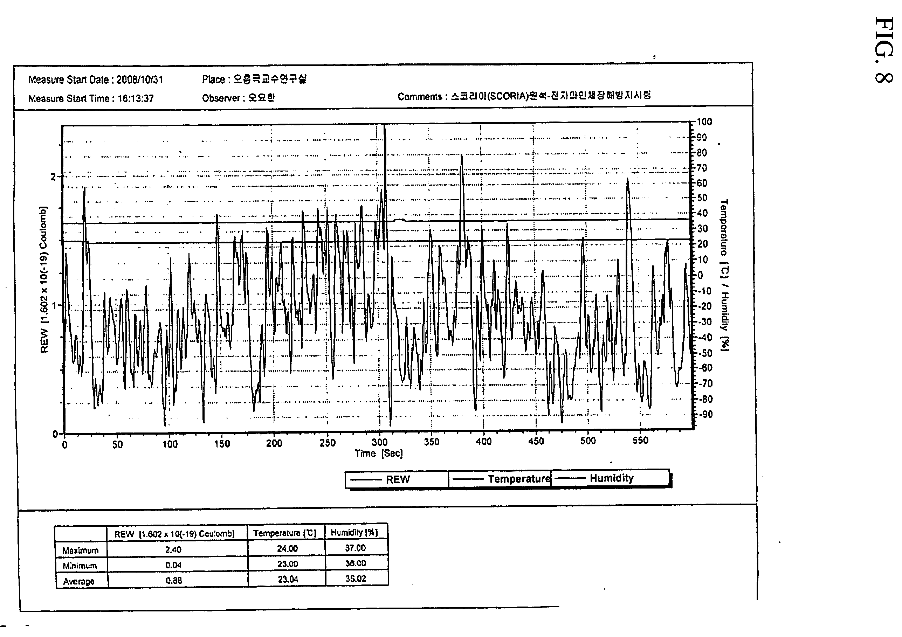

FIG. 8 shows the results of the test of the effect of scoria rock for preventing the influence of electromagnetic waves to human body, in which harmful left-hand energy of electromagnetic waves emitted from cellular phones, radios, etc. is removed by right-hand energy, from which it can be seen that the effect of scoria rock for preventing the influence of electromagnetic waves to human body is excellent. In particular, according to some medical reports, it is reported that the most significant cause of cancer is electromagnetic waves. Therefore, it is considered that the effect of scoria rock for preventing the influence of electromagnetic waves to human body can provide the effect of preventing cancer caused by electromagnetic waves.

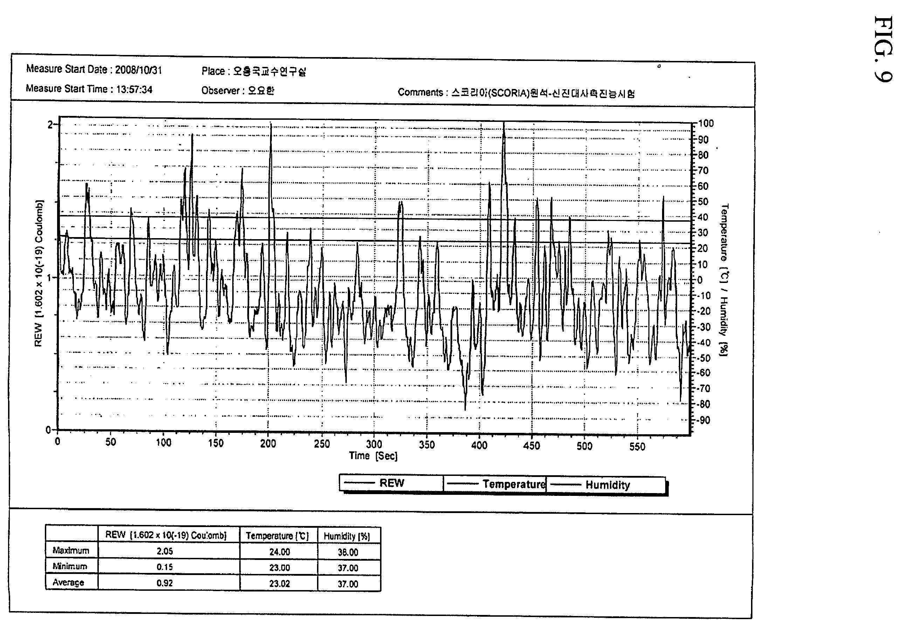

FIG. 9 shows the results of the test of the effect of scoria rock for promoting metabolism, in which the crossed vibration states of left-hand energy and right-hand energy emitted from measurement samples are compared with the vibration state of human body energy, from which it can be seen that the effect of scoria rock for promoting metabolism is excellent.

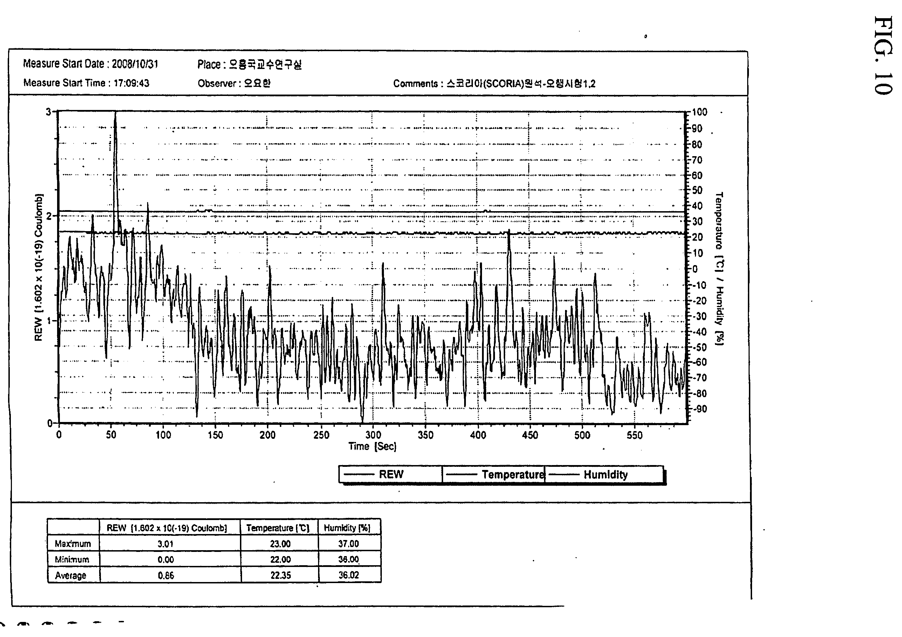

FIGS. 10 to 12 show the results of the measurement of five primary substances (wood, fire, earth, metal, and water) of scoria rock, from which it can be seen that the scoria rock is turned out to be fire.

Meanwhile, when the scoria is brought into contact with the body, the above-described effects of scoria can be more effectively provided to a user. That is, the fabric provided with the scoria molded body according to the present invention may be manufactured in various forms of fabric products worn on the human body such as underwear, socks, hats, gloves, belts, etc. Preferably, the fabric may be manufactured in the form of underwear that is in direct contact with the user's body. Here, the first fabric sheet 110 and the second fabric sheet 120 between which the scoria molded body 130 is provided may be a lining and an outer fabric of a fabric product. Moreover, the scoria molded body 130 may be provided in the entire or any portion of the fabric product according to the present invention.

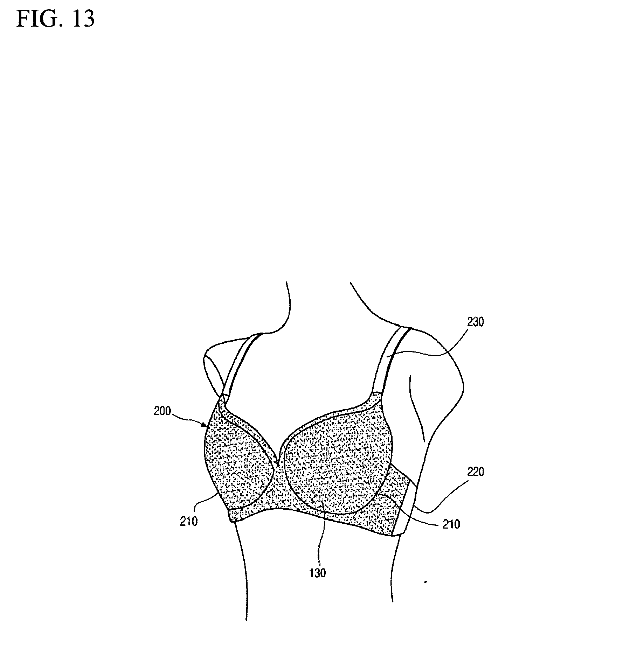

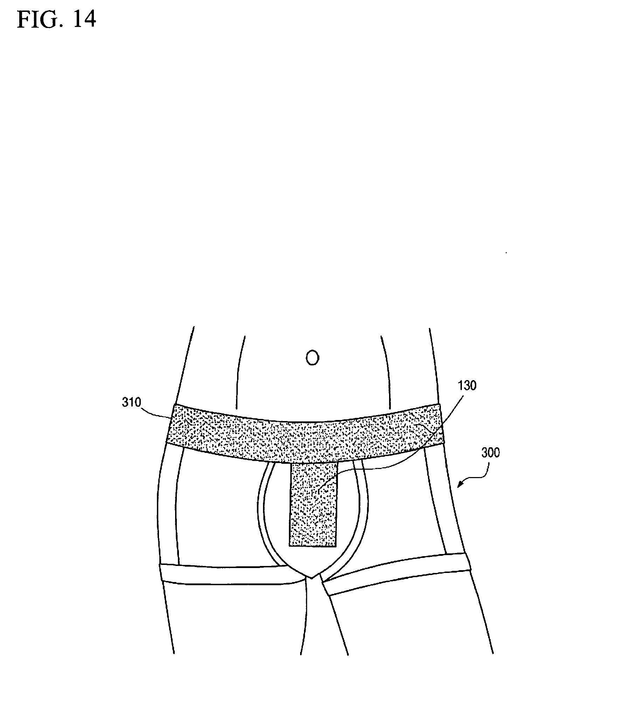

FIGS. 13 and 14 are diagrams illustrating the fabric product provided with the scoria molded body according to the present invention, in which a brassiere 200 and a pair of briefs 300 are shown. Here, the scoria molded body 130 may preferably be disposed on portions corresponding to the front and rear of the body to more effectively provide the rotating electromagnetic wave characteristics to the human body.

As shown in FIG. 13, the scoria molded body 130 is disposed between a lining and an outer fabric of a brassiere 200. The brassiere 200 comprises a pair of brassiere cups 210 connected to each other to be fitted to a woman's breast, a brassiere strap 220 attached to both ends of the brassiere cups 210 and connected to each other by a connection means at the back of the body, and a pair of shoulder straps 230 for maintaining the brassiere cups 210 so as not to descend. Here, the scoria molded body 130 is disposed at the brassiere cups 210 and the brassiere strap 220 to be disposed at the front and rear of the body. The scoria molded body 130 may be disposed in the entire brassiere strap 220 or may not be disposed on both sides of the brassiere strap 220 as shown in FIG. 13.

Especially, when the user wears the brassier 200, the scoria molded body 130 disposed in the brassiere cups 210 subtly vibrates the cells in the user's breast due to far-infrared ray radiation characteristics to activate the cells, thereby compensating for poor breast. Moreover, the scoria molded body 130 promotes immunization activities and biological activities of the body due to rotating electromagnetic wave characteristics such that the user can be protected from various adult diseases including cancer by a simple way of wearing clothes.

As shown in FIG. 14, the scoria molded body 130 may be disposed between a lining and an outer fabric of a pair of briefs 300. The briefs 300 comprise a waist band 310 and a fabric 320. Here, the scoria molded body 130 is disposed on the waist band 310 and a perineal region of the fabric 320 of the briefs 300 to be disposed at the front and rear of the body.

Meanwhile, although the scoria molded body 130 is disposed between the lining and the outer fabric of the fabric product worn directly on the user's body, such as brassiere, briefs, etc., in FIGS. 13 and 14, the fabric 100 provided with the scoria molded body 130 as shown in FIG. 1 may be attached to a wear product worn on the body when the fabric product comprises a single layer or a non-fabric material.

That is, in the present invention the fabric 100 provided with the scoria molded body 130 as shown in FIG. 1 may be separately attached to a wear product worn on the body, for example, underwear such as brassiere, briefs, etc., bracelet, band, belt, etc. Here, the fabric 100 provided with the scoria molded body 130 may be attached to a wear product using an adhesive or by sewing depending on the material of the wear product.

Moreover, the fabric 100 provided with the scoria molded body 130 may be attached between the lining and the outer fabric of the wear product or attached to an appropriate portion of the inside or outside of the wear product.

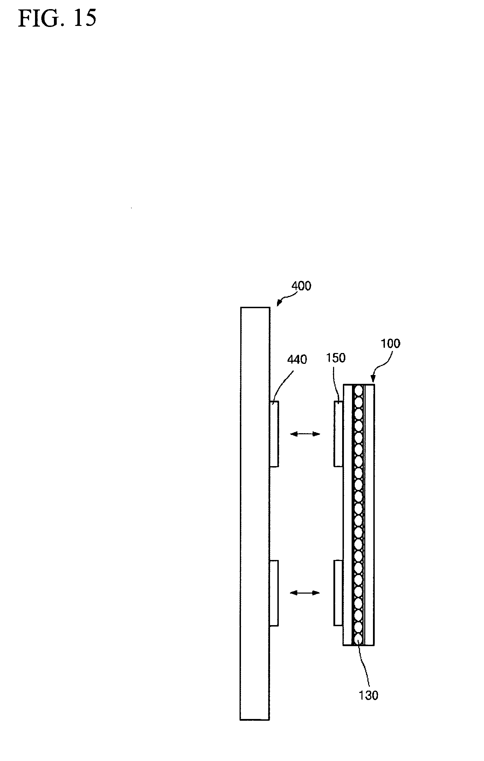

Furthermore, the fabric 100 provided with the scoria molded body 130 may be attached to the wear product by the following method. As shown in FIG. 15, a first fastening means 440 is provided on an inner side of a wear product 400 such as brassiere, briefs, etc., a second fastening means 150 corresponding to the first fastening means 440 is provided on one side of the fabric 100 provided with the scoria molded body such that the fabric 100 provided with the scoria molded body can be attached to and detached from the wear product 400. Here, the first and second fastening means 440 and 150 may be various detachable fastening means such as Velcro tape, snap button, etc.

That is, according to the exemplary embodiments, the user can easily experience far-infrared radiation effects and rotating electromagnetic wave effects of scoria in such a manner than the scoria molded body is brought into contact with the user's body.

Meanwhile, although the scoria is attached to the fabric by forming the scoria molded body and attaching the scoria molded body to the fabric using an adhesive in the exemplary embodiments, the scoria may be attached to the fabric in the following manner. That is, a scoria rock is crushed into scoria powder, the scoria powder is mixed with an adhesive to prepare a mixed solution, and the mixed solution is attached to a desired portion of the fabric water in a desired form by screen printing, for example. Here the adhesive may be a silicone adhesive.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents.

## Claims

1. A fabric comprising a scoria molded body disposed between a first fabric sheet and a second fabric sheet,
wherein the scoria molded body is formed by mixing scoria powder containing silicon oxide and titanium oxide with water and molding and solidifying the mixture and has a far-infrared radiation rate of 90% or higher and rotating electromagnetic wave characteristics.

2. The fabric of claim 1, wherein the fabric is attached to a wear product worn on a human body.

3. The fabric of claim 2, wherein the fabric is attached to and detached from the wear product.

4. A fabric product comprising a scoria molded body disposed between a lining and an outer fabric,
wherein the scoria molded body is formed by mixing scoria powder containing silicon oxide and titanium oxide with water and molding and solidifying the mixture, has a far-infrared radiation rate of 90% or higher and rotating electromagnetic wave characteristics, and is disposed on portions corresponding to the front and rear of a human body.

5. A fabric product comprising a scoria molded body disposed between a lining and an outer fabric of a brassiere,
wherein the scoria molded body is formed by mixing scoria powder containing silicon oxide and titanium oxide with water and molding and solidifying the mixture and has a far-infrared radiation rate of 90% or higher and rotating electromagnetic wave characteristics.

6. The fabric product of claim 5, wherein the scoria molded body is disposed on a pair of brassiere cups and a brassiere strap.

7. A fabric product comprising a scoria molded body disposed between a lining and an outer fabric of a pair of briefs,
wherein the scoria molded body is formed by mixing scoria powder containing silicon oxide and titanium oxide with water and molding and solidifying the mixture and has a far-infrared radiation rate of 90% or higher and rotating electromagnetic wave characteristics.

8. The fabric product of claim 7, wherein the scoria molded body is disposed on a waist band and a perineal region.

9. A method for adhering scoria, the method comprising:
a first step of preparing a mixed solution by mixing scoria powder, which contains silicon oxide and titanium oxide with and has rotating electromagnetic wave characteristics, with an adhesive; and
a second step of applying the mixed solution onto a fabric.

10. The method of claim 9, wherein in the second step, the mixed solution is applied to the fabric by screen printing.
